**Europäisches Patentamt**

**European Patent Office**

**Office européen des brevets**

(19)

(11) **EP 1 210 952 B1**

(12) **EUROPEAN PATENT SPECIFICATION**

(45) Date of publication and mention
of the grant of the patent:
**27.09.2006 Bulletin 2006/39**

(51) Int Cl.:
*A61L 2/20* *(2006.01)*   *A61L 2/14* *(2006.01)*
*A61L 2/18* *(2006.01)*   *B01D 1/00* *(2006.01)*

(21) Application number: **01310116.7**

(22) Date of filing: **03.12.2001**

(54) **Sterilant vaporizer**

Sterilisierungsmittelverdampfer

Evaporateur d'agent stérilisant

(84) Designated Contracting States:
**DE ES FR GB IT**

(30) Priority:   **04.12.2000   US 728973**

(43) Date of publication of application:
**05.06.2002   Bulletin 2002/23**

(73) Proprietor: **ETHICON, INC.**
**Somerville,**
**New Jersey 08876 (US)**

(72) Inventors:
• **Nguyen, Nick N.**
**Costa Mesa, CA 92626 (US)**

• **Kohler, James P.**
**Laguna Hills, CA 92653 (US)**

(74) Representative: **Fisher, Adrian John et al**
**CARPMAELS & RANSFORD**
**43-45 Bloomsbury Square**
**London WC1A 2RA (GB)**

(56) References cited:
**EP-A- 0 321 908**      **WO-A-97/47331**
**DE-A- 2 639 301**      **US-A- 6 106 772**

**Description**

**FIELD OF THE INVENTION**

The present invention relates to vaporizers and methods of vaporizing, and more particularly to vaporizers for chemical vapor sterilization systems and for a method of vaporizing with sterilants.

**BACKGROUND**

Vapor based chemical sterilization systems are a popular alternative to steam sterilization. They typically allow sterilization at lower temperatures than is possible with steam, thereby allowing sterilization of articles sensitive to high temperatures. Several such systems are commercially available, such as the STERRAD Brand hydrogen peroxide gas/ plasma sterilization system.

In this system, a sterilization chamber is brought to low pressures, approximately one Torr, and liquid hydrogen peroxide is admitted into the chamber and vaporized into the low pressure. The hydrogen peroxide vapor diffuses to articles placed within the chamber. After a time, an electromagnetic field or other means is employed to ignite a plasma of the hydrogen peroxide vapor and after the plasma inducing field is removed, the constituents reassemble to form oxygen and water. Such systems are more fully described in U.S.-A-4,643,876 and US-A-4,756,882..

Solutions of hydrogen peroxide and other liquid sterilants typically contain non-vaporizable constituents; for instance, in a typical 59% concentration hydrogen peroxide solution, trace quantities of chemicals such as transition metal salts and organic free radical scavengers, are present to stabilize the liquid solution. Upon vaporization of the hydrogen peroxide solution, these chemicals are left as solid particulates. If no effort is made to separate and collect these constituents, they may become deposited upon items in the sterilization chamber. Most of these constituents are harmless, and their presence is merely unsightly and/or perhaps provides a false impression that these sterilization processes were not complete. However, in some sterilization processes, these constitutes may either be harmful to the instruments and to the patient. Accordingly, it is desirable to remove such constituents prior to releasing the vaporized hydrogen peroxide or other sterilant to the sterilization chamber

U.S.-A-6,106,772 addresses this problem by providing an impingement plate outside of the vaporizer in the chamber upon which the stream of hydrogen peroxide which is being vaporized impinges prior to the contacting the devices or load to be sterilized in the sterilization chamber. In this fashion, a portion of the non-vaporizable constituents adheres to the plate rather than depositing onto the load in the sterilization chamber. While such system provides a marked improvement over no control of non-vaporizable constituents, small amount of such non-vaporizable constituents may still deposit on the load in the sterilization chamber. Accordingly, it would be desirable to provide a system and method for collecting such constituents with a higher degree of efficiency.

The STERRAD 200 brand hydrogen peroxide/gas plasma type sterilizer employs a vaporizer in which the vaporizing hydrogen peroxide follows a path formed by a series of annular fins in a cylindrical chamber creating a series of torus-like spaces, and wherein each fin has an opening therethrough offset from the opening in the adjacent fins whereby to provide a series of direction changes through the vaporizer.

**SUMMARY OF THE INVENTION**

The present applicants have discovered that by providing a flow restriction, residence time within the vaporizer is enhanced and the efficiency of the vaporizer is also enhanced.

A vaporizer according to the present invention vaporizes a sterilant from its liquid phase in a vapor phase sterilization system having a pressure below atmospheric pressure. The vaporizer comprises an inlet to receive the sterilant in its liquid phase, an outlet to discharge the sterilant in its vapor phase, a circuitous path between the inlet and the outlet to collect non-vaporizable ingredients of the sterilant, and a flow restriction.

Preferably, the circuitous path comprises a plurality of baffles. The circuitous path can comprise an inner tube positioned concentrically within an outer tube, the circuitous path including a first portion in a first direction between the inner tube and the outer tube and a second portion in a second opposite direction through the inner tube. The circuitous path comprises at least one portion in which an effective cross-sectional area of the portion increases by at least 89% to decrease the speed of the sterilant passing therethrough. The circuitous path preferably comprises at least two turns, each of which are at least 90 degrees.

The flow restriction can comprise an orifice having a cross-sectional area no greater than 44.1% of a cross-sectional area of the circuitous path immediately upstream of the orifice. Restriction retains the vapor within the vaporizer for at least 17 milliseconds, and more preferably for at least 26 milliseconds.

A method of providing a vapor phase sterilant to a sterilization chamber, according to the present invention, comprising the steps of creating temperature and pressure conditions within a vaporizer sufficient to vaporize the sterilant and admitting the sterilant, in its liquid phase, into the vaporizer and vaporizing the sterilant. The sterilant passes through a circuitous path where non-vaporizable components of the sterilant collect on surfaces forming the circuitous path. The sterilant, in its vapor phase, passes through a flow restriction which increases residency within the circuitous path and enhances efficiency of collecting non-vaporizable components. The vaporized sterilant passes out of the vaporizer.

The non-vaporizable components can comprise stabilizing compounds for the liquid phase of the sterilant. The sterilant can comprise hydrogen peroxide.

Preferably, at least 50%, and more preferably at least 75%, of the non-vaporizable components are removed from the sterilant prior to the step of passing the sterilant out of the vaporizer.

## BRIEF DESCRIPTION OF THE EDRAWINGS

FIG. 1 is a flow diagram of a sterilization system employing a vaporizer according to the present invention;
FIG. 2 is a perspective view of a first embodiment of the vaporizer of FIG. 1;
FIG. 3 is an exploded sectional view taken along lines 3 - - 3 of FIG. 2, in which the core is partially removed;
FIG. 4 is an exploded sectional view taken along lines 3 - - 3 of FIG. 2, in which the core is not removed;
FIG. 5 is a perspective sectional view taken along lines 3 - - 3 of FIG. 2;
FIG. 6 is a sectional view of a second embodiment of a vaporizer according to the present invention;
FIG. 7 is a sectional view of a third embodiment of a vaporizer according to the present invention;
FIG. 8 is a sectional view of a fourth embodiment of a vaporizer according to the present invention;
FIG. 9 is a sectional view of an outlet tube of a fifth embodiment of a vaporizer according to the present invention;
FIG. 10 is a perspective view of the system of FIG. 1; and
FIG. 11 is a side elevation view of the system of FIG. 10.

## DESCRIPTION

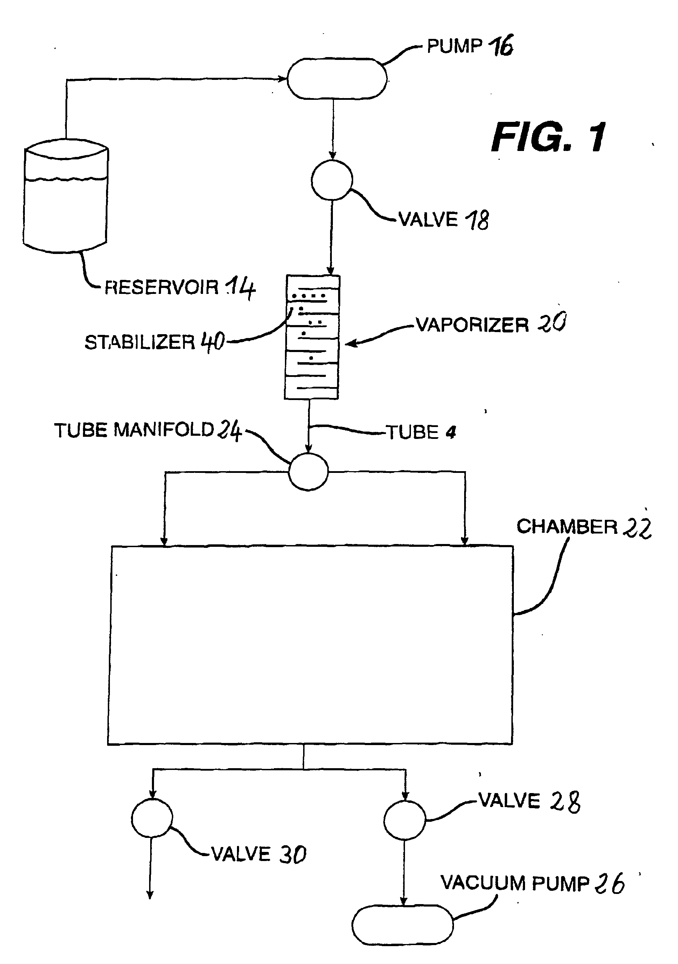

FIG. 1 illustrates in schematic format of a vapor phase sterilization system 10 and components for providing sterilant thereto. Liquid sterilant, such as a 59% solution of hydrogen peroxide and water, is stored within a reservoir 14. A pump 16 and valve 18 control flow of sterilant 12 from the reservoir 14 to a vaporizer 20. The vaporizer 20 connects to a sterilization chamber 22 through a manifold 24. A vacuum pump 26 and a valve 28 provide means for drawing a vacuum on the chamber 22 and a vent valve 30 allow venting of the chamber 22 to atmosphere.

Before admission of the sterilant 12, a vacuum is drawn on the chamber 22 by the vacuum pump 26. Typically, the vacuum is approximately 1 Torr. The vaporizer 20 is fluidly connected to the chamber 22 and is, therefore, effectively at the same pressure initially as the chamber 22 with the exception of the flow induced pressure drops therebetween. Liquid sterilant 12 enters the vaporizer through an inlet 32 and immediately begins vaporizing due to the low pressure and heated vaporizer therein.

It travels a circuitous path 34 therethrough, such as created by a series of baffles 36 or other flow direction changing objects which provide a plurality of directional changes, thereby allowing the flow of vaporizing sterilant 12 to impinge upon surfaces 38 with the vaporizer 20 as it passes therethrough. Such impingement causes non-vaporizable components 40 in the sterilant 12 to deposit upon these impingement surfaces 38. As a sterilant 12 exits the vaporizer 20 through its exit 42, a fairly large proportion of the non-vaporizable components 40 are left adhered to the impingement surfaces 38 within the vaporizer. Thus, as the sterilant 12 travels through the manifold 24 into the chamber 22 it is relatively free of non-vaporizable constituents.

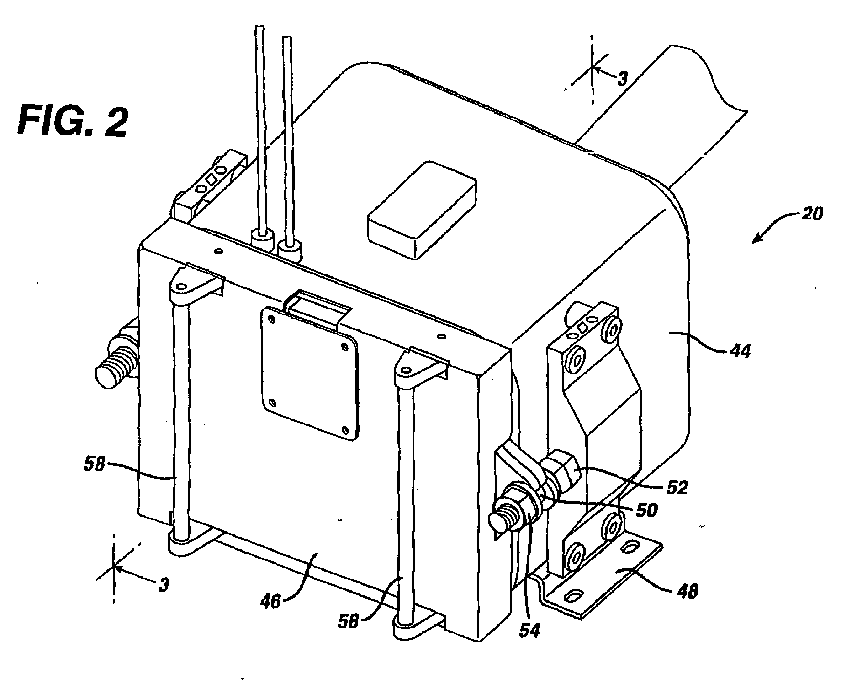

FIG. 2 shows one embodiment of the vaporizer 20 according to the present invention. It comprises a housing 44 having a removable panel 46. The housing 44 fits into a mounting bracket 48. Threaded fittings 50 on the bracket 48 connect to lugs 52 and 54 on the housing 44 and panel 46 respectively and are held by means of nuts 56. Handles 58 are provided on the panel 46 for removing the panel.

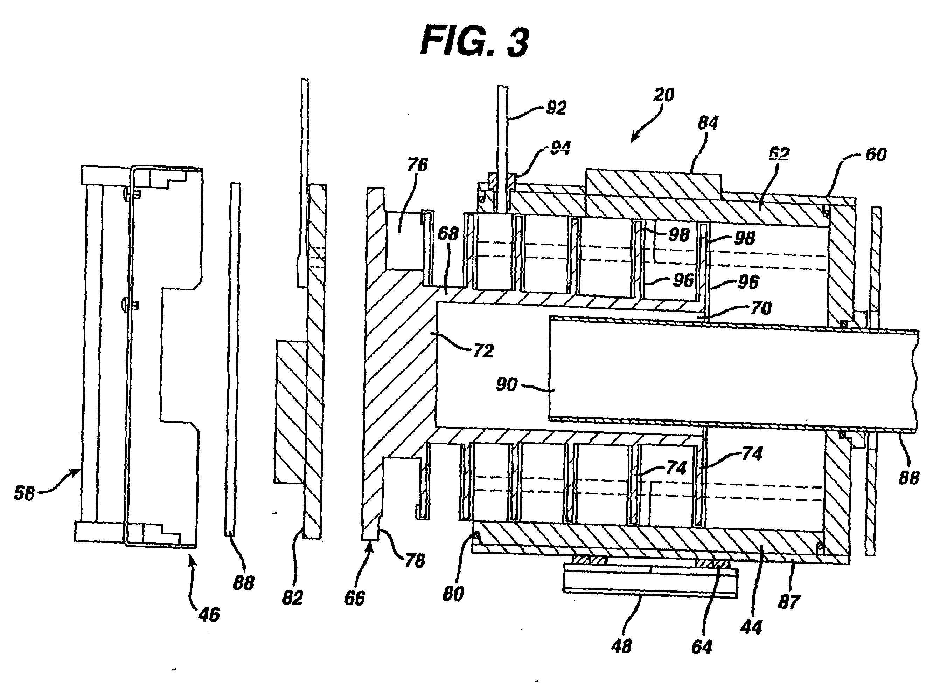

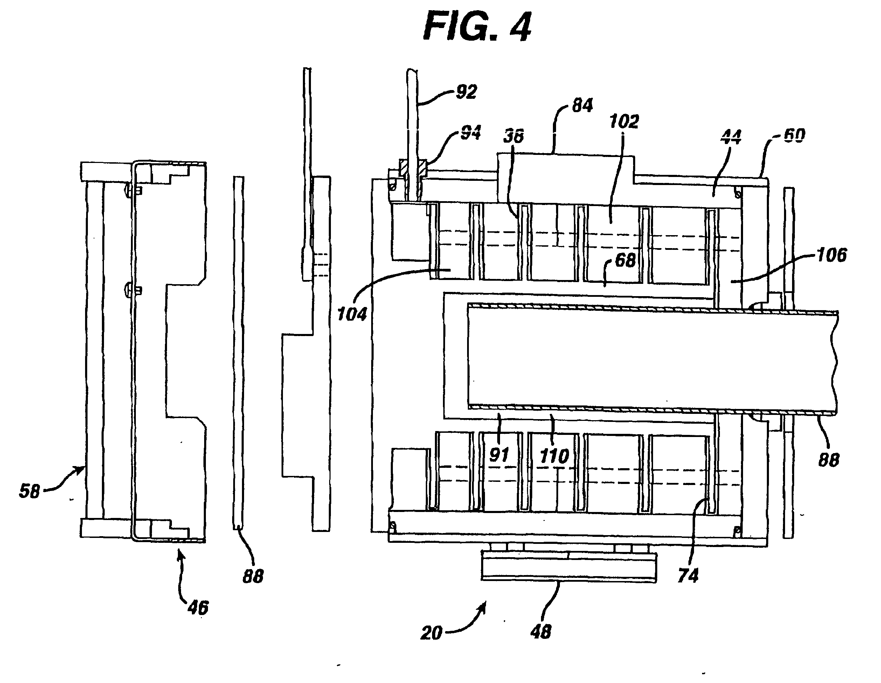

Turning also to FIGS. 3 and 4, it can be seen that the entire housing 44 is insulated by a blanket 60, which may comprise any suitable insulation. An electric heater 62 lies between the blanket 60 and the housing 44. The spacers 64 between the housing 44 and the mounting bracket 48 also help to reduce heat loss from the housing 44.

A core 66 fits within the housing 44. The core 66 comprises a cylinder 68 having an open end 70 and closed end 72 with a plurality of annular fins 74 extending radially therefrom. The fins 74 extend toward the housing 44 but do not actually touch the housing. A partition 76 having an annular lip 78 attaches to the cylinder closed end 72 and seals against the housing 44 by means of an O-ring 80. A core heater 82 having a thermostat 84 and thermister 86 attached to the partition 76 to heat the core 66. An insulating blanket 87 covers the heater 82. All of this is enclosed by the removable panel 46 so that the core 66 can be easily removed for cleaning.

The core cylinder 68 fits over an outlet tube 88 which extends into the housing 44. The outlet tube 88 has an outside diameter slightly smaller than the inside diameter of the core cylinder 68 and has an open end 90 which sits adjacent to but does not abut the cylinder closed end 72. The tight fit between the outlet tube 88 and core cylinder 68 creates a flow restriction 91.

A pair of liquid tubes 92 enter the housing 44 adjacent the partition 76 and are preferably attached through a fitting 94.

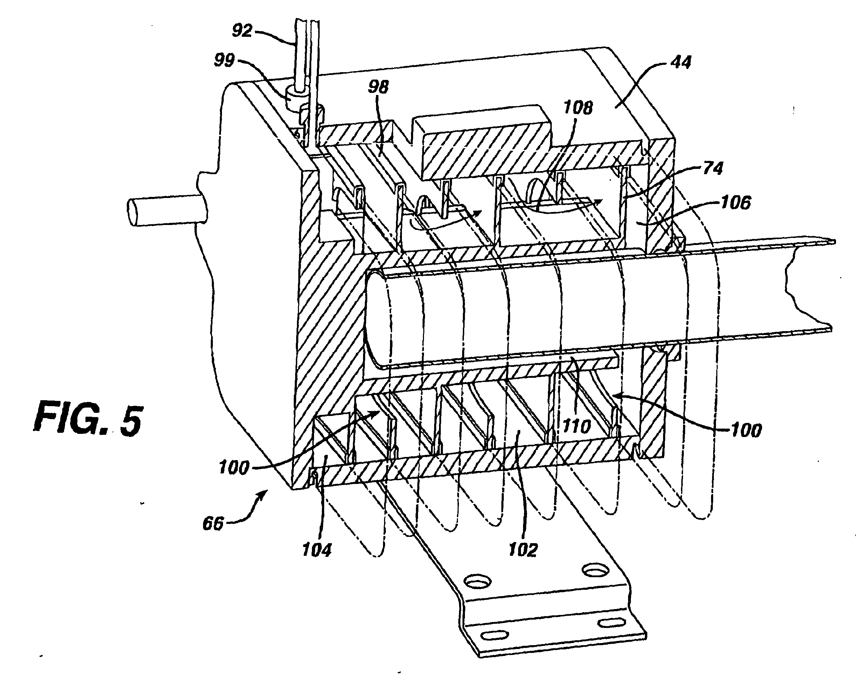

Turning also now to FIG. 5, a gasket 96 covers distal edges 98 of each of the fins 74 to seal the fins 74 against the housing 44. A series of openings 100 through the fins adjacent the cylinder 68 are provided and are offset from each other on adjoining fins 74 so that the gases may flow past the fins 74 through the openings 100, but in doing so make frequent directional changes. The fins 74 create a series of spaces or pockets 102 with an inlet pocket 104 adjacent the liquid tubes 92 and a terminal pocket 106 adjacent the cylinder open end 70. Liquid entering the vaporizer 20 through the liquid tubes 92 is vaporized and flows along a circuitous path 108 through the openings 100 to the terminal pocket 106 and then into a space 110 between the cylinder 68 and outlet tube 88. It enters the space 110 through the cylinder open end 70. Flow then proceeds into the outlet tube 88 through its open end 90. Along the way, such flow impinges upon many surfaces leaving behind deposits of non-vaporizable components 40.

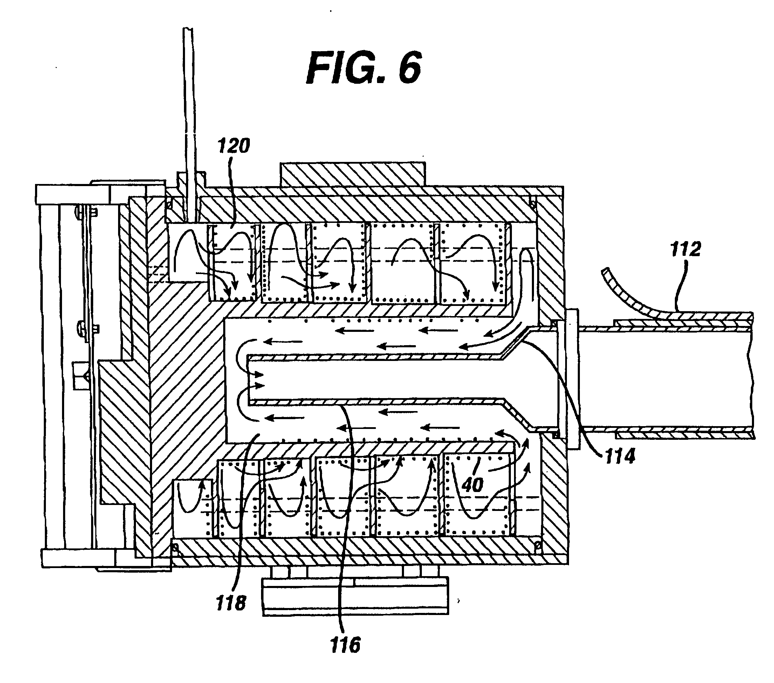

FIG. 6 illustrates an alternative embodiment of the invention. This embodiment employs an outlet tube 112 having a reducing section 114 at the interface with the housing 44 and which leads to a smaller diameter section 116 within the cylinder 68. Accordingly, a space 118 between the outlet tube 112 and cylinder 68 has a larger cross-sectional area than in the previous embodiment. This reduces the velocity of the flow in the space 118 and increases residence time so as to allow a higher portion of the non-vaporizable components 40 to come out of the sterilant 12. The narrow diameter of the small diameter section 116 also enhances this effect as the cross-sectional area in the small diameter section 116 is less than the cross-sectional area in the space 118 or in a space 120 between the cylinder 68 and housing 44 thereby acting as a flow restriction.

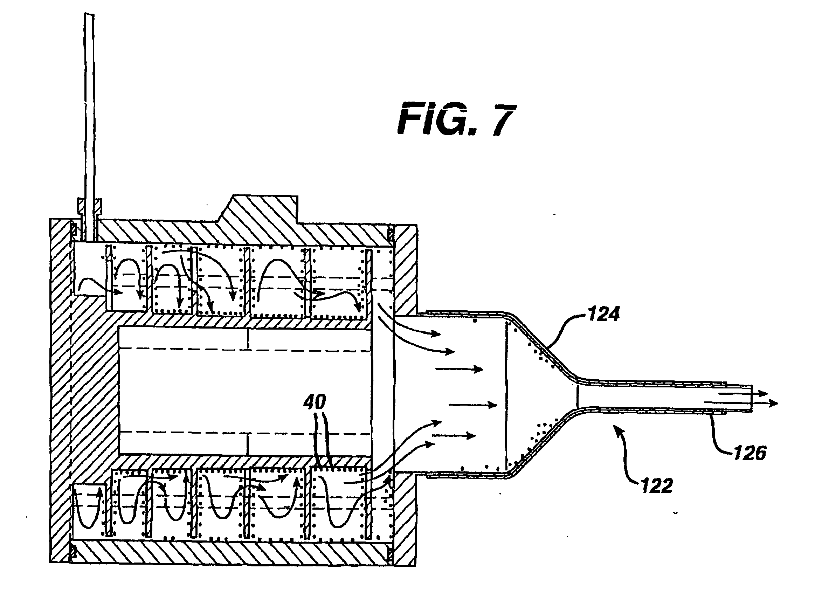

FIG. 7 illustrates a further embodiment in which an outlet tube 122 does not extend into the housing but has a high area ratio (greater than or equal to 3:1) reducing section 124 leading to a very narrow outlet portion 126 thereby providing a flow restriction. This large flow restriction substantially decreases the velocity in the remainder vaporizer 20 thereby allowing a longer residence time and a higher degree of separation of the non-vaporizable components 40 for a given size of the vaporizer 20. Alternatively, the size of the vaporizer 20 can be reduced while maintaining the same level of efficiency in removing non-vaporizable components 40.

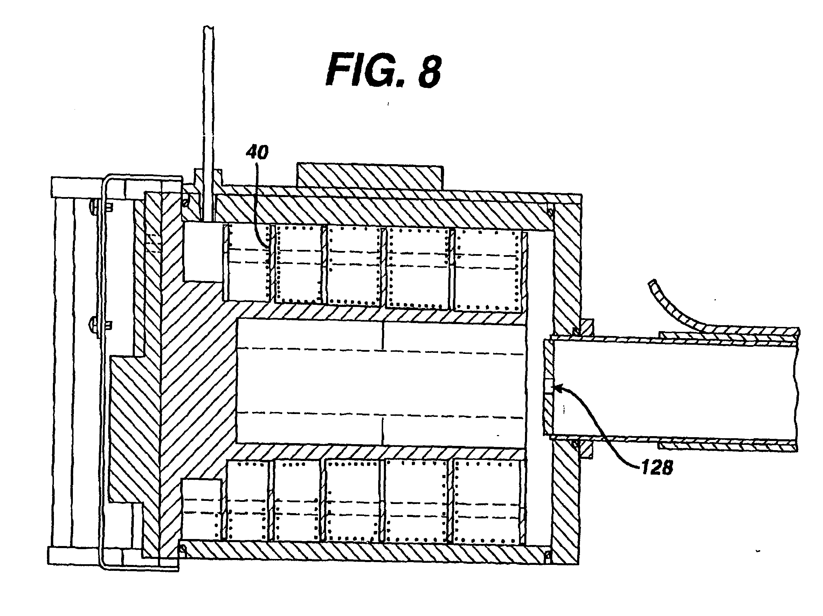

FIG. 8 illustrates the same concept but employs an orifice 128 rather than a reducing section to provide a flow restriction.

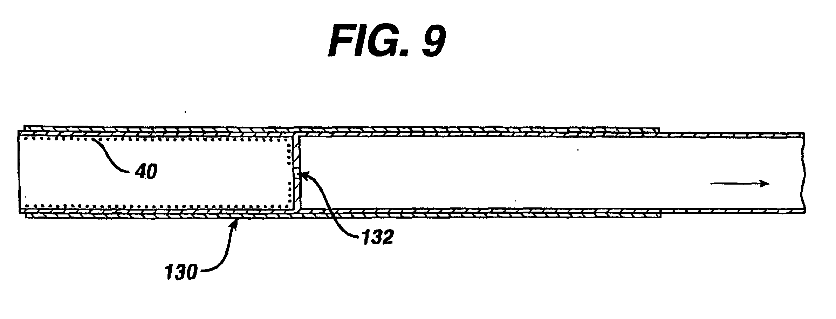

FIG. 9 illustrates an outlet tube 130 having an orifice 132 in the middle thereof.

TABLE 1 illustrates how a flow restriction can enhance the efficiency of the vaporizer 20 in collecting the non-vaporizable components 40. It illustrates the performance difference of a vaporizer configured according to that shown in FIG. 6 having two different size small diameter sections 116. By reducing the diameter by 50%, the collection efficiency was increased from 76% to 100%.

TABLE 1

FLUID VELOCITY AND RESIDENCE TIME IN THE VAPORIZER AT 70°C WITH TWO DIFFERENT SIZES OF OUTLET TUBES INJECTION OF 15 ML OF 59 WT% HYDROGEN PEROXIDE SOLUTION

| 0.75 in OD Tube | | | | |
|---|---|---|---|---|
| Space Number | Cross Sectional Area in$^2$ | Average Velocity ft/sec | Stabilizer Collected, g | Residence Time, milliseconds |
| 120 | 1.8 | 158 | 2.67 | 26 |
| 118 | 4.7 | 59 | 0.06 | |
| 116 | 0.4 | 747 | 0 | |
| Total Collected, g | | | 2.73 | |
| Measured from Solution, g | | | 2.60 | |
| % Recovered | | | 100 | |
| Ratio of (space 116 / space 118) = 0.4/4.7 = 8.5% | | | | |
| Ratio of (space 118 - space 120) / (space 120) = 161% | | | | |

(continued)

### 1.5 in OD Tube

| Space Number | Cross Sectional Area in$^2$ | Average Velocity ft/sec | Stabilizer Collected, g | Residence Time, milliseconds |
|---|---|---|---|---|
| 120 | 1.8 | 245 | 1.96 | 17 |
| 118 | 3.4 | 127 | 0.02 | |
| 116 | 1.5 | 286 | 0 | |
| Total Collected, g | | | 1.98 | |
| Measured from Solution, g | | | 2.60 | |
| % Recovered | | | 76(75% in Space 120) | |
| Ratio of (space 116 / space 118) = 1.5/3.4 = 44.1% | | | | |
| Ratio of (space 118 - space 120) / (space 120) = 89% | | | | |
| 1 in $\hat{=}$ 2,54 cm | | | | |
| 1 in$^2$ $\hat{=}$ 6,45 cm$^2$ | | | | |
| 1 ft/sec $\hat{=}$ 0,3048 m/sec | | | | |

The residence time of vapor retained in the vaporizer can be calculated according to the following equations:

$$t = (L/v) \times 1000,$$

$$v = (W \times 144) / (\rho \times A),$$

1 ft $\hat{=}$ 0,3048 m
1 ft$^2$ $\hat{=}$ 6,45 cm$^2$
1 ft$^3$ $\hat{=}$ 0,02832 m$^3$
1 psia $\hat{=}$ 16,89 KPa
1 lb/sec $\hat{=}$ 0,4536 kg/sec
1 lb/ft$^3$ $\hat{=}$ 16,018 kg/m$^3$

where

t = calculated residence time, milliseconds,
L = measured length of flow path, ft,
v = calculated vapor velocity, ft/sec,
w = measured mass flow rate, lb/sec,
$\rho$ = calculated vapor density, (P x MW)/(R x T), lb/ft$^3$,
P = measured upstream pressure in vaporizer, psia,
MW = calculated vapor molecular weight, g/mole,
R = gas constant, mmHg-l/mole °K,
T = measured vapor temperature, °K,
A = measured cross sectional area for flow in the vaporizer, in$^2$.

The 17 milliseconds residence time for the 1.5 inches OD tube can be calculated with the follow measured data.

$$L = 4.1 \text{ ft}$$

$$W = 1.4 \times 10^{-3} \text{ lb/sec}$$

$$P = 0.125\ \mathrm{lb_f/in^2}$$

$$T = 343\ ^{\circ}K$$

$$A = 1.75\ \mathrm{in^2}$$

1 lb/in$^2$ $\hat{=}$ 0,0703 kg/m$^2$

$$\rho = (P \times MW) / (R \times T)$$
$$= (0.125\ \mathrm{lb_f/in^2} \times 760\ \mathrm{mmHg/atm} \times 25\ \mathrm{g/mole} \times 28.32\ \mathrm{l/ft^3}) / (14.7\ \mathrm{lb_f/in^2\text{-}atm} \times 62.36\ \mathrm{mmHg\text{-}l/mole\ ^{\circ}K} \times 343\ ^{\circ}K \times 454\ \mathrm{g/lb})$$
$$= 4.7 \times 10^{-4}\ \mathrm{lb/ft^3}$$

1 atm $\hat{=}$ 101,33 KPa
1 mm Hg $\hat{=}$ 1,33 KPa

$$v = W/(\rho A)$$
$$= (1.4 \times 10^{-3}\ \mathrm{lb/sec} \times 144\ \mathrm{in^2/ft^2}) / (4.7 \times 10^{-4}\ \mathrm{lb/ft^3} \times 1.75\ \mathrm{in^2})$$
$$= 245\ \mathrm{ft/sec}$$

$$t = (L/v) \times 1000$$
$$= 4.1\ \mathrm{ft} \times 1000\ \mathrm{milliseconds/sec} / 245\ \mathrm{ft/sec} = 17\ \mathrm{milliseconds}$$

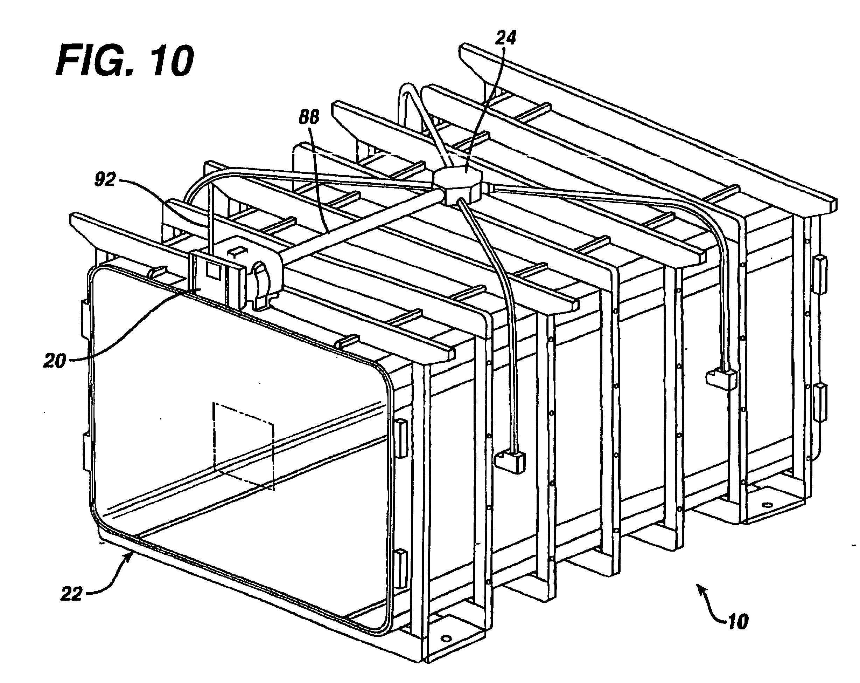

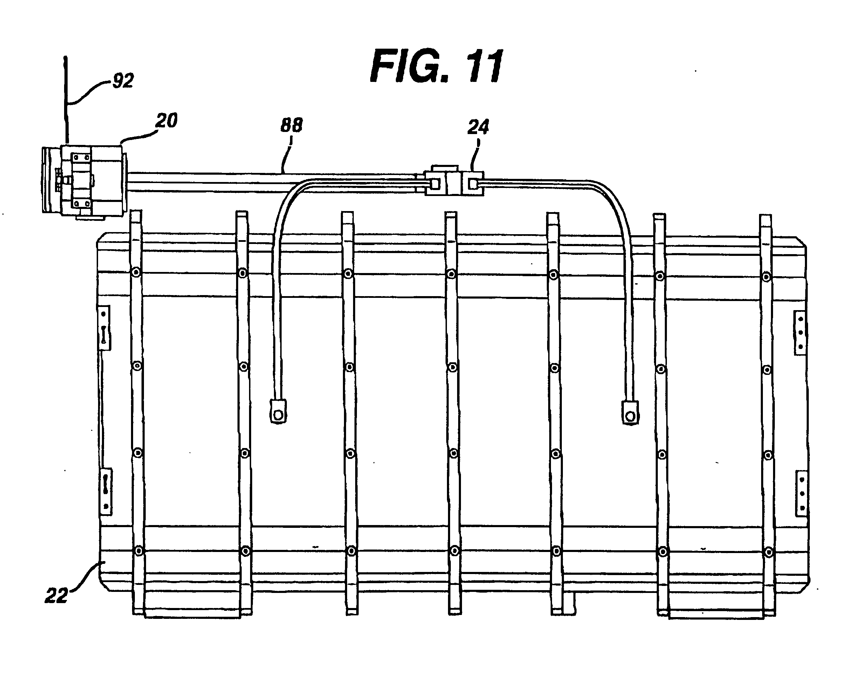

FIGS. 10 and 11 illustrate the system 10 with the vaporizer 20 located atop the sterilizer chamber 22 and showing the manifold 24 leading from the vaporizer 20 into various locations into the sterilization chamber.

After a number of cycles, a sufficient amount of non-vaporizable components 40 will become deposited on the components within the vaporizer 20 and it will be desirable to remove these deposits. Preferably, the housing 44 tapers slightly from where the panel 46 attaches to where the outlet tube 88 leaves so that when the panel 46 is removed the core 66 can be slid out of the housing 44 more easily. If it becomes stuck, the nuts 56 can be turned to drive the core 66 out of the housing 44.

The invention now being fully described, it will be apparent to one of ordinary skill in the art that many modifications and changes can be made thereto without departing from the scope of the invention as defined in the following claims.

## Claims

1.   A vaporizer (20) for vaporizing a sterilant from its liquid phase in a vapor phase sterilization system having a pressure below atmospheric pressure, said vaporizer comprising:

  an inlet (92) whereby to receive the sterilant in its liquid phase;
  an outlet (88) whereby to discharge the sterilant in its vapor phase;
  a circuitous path between the inlet and the outlet whereby to collect non-vaporizable ingredients of the sterilant; and
  a flow restriction (91, 116, 126, 128, 132) between the circuitous path and the outlet, wherein the restriction can retain the vapor within the vaporizer for at least 17 milliseconds.

**2.** A vaporizer according to claim 1 wherein the circuitous path comprises a plurality of baffles (74).

**3.** A vaporizer according to claim 1 wherein the circuitous path comprises an inner tube (88, 116) positioned concentrically within an outer tube (68), the circuitous path including a first portion in a first direction between the inner tube and the outer tube and a second portion in a second opposite direction through the inner tube.

**4.** A vaporizer according to claim 1 wherein the circuitous path comprises at least one portion in which an effective cross-sectional area of the portion increases by at least 89% whereby to decrease the speed of the sterilant passing therethrough.

**5.** A vaporizer according to claim 1 wherein the flow restriction comprises an orifice (128, 132) having a cross-sectional area no greater than 44.1% of a cross-sectional area of the circuitous path immediately upstream of the orifice.

**6.** A vaporizer according to claim 1 wherein the circuitous path comprises at least two turns, each of which are at least 90 degrees.

**7.** A vaporizer according to claim 1 wherein the restriction can retain the vapor within the vaporizer for at least 26 milliseconds.

**8.** A method of providing a vapor phase sterilant to a sterilization chamber comprising the steps of:

creating temperature and pressure conditions within a vaporizer sufficient to vaporize the sterilant;
admitting the sterilant, in its liquid phase, into the vaporizer and vaporizing the sterilant;,
passing the sterilant through a circuitous path and collecting non-vaporizable components of the sterilant on surfaces forming the circuitous path;
parsing the sterilant, in its vapor phase, through a flow restriction; and
passing the sterilant, in its vapor phase, out of the vaporizer.

**9.** A method according to claim 8 wherein the step of passing the sterilant through a circuitous path comprises passing the sterilant past a plurality of baffles.

**10.** A method according to claim 8 wherein the step of passing the sterilant through the circuitous path comprises passing the sterilant in a first direction through an inner tube positioned concentrically within an outer tube and in a second opposite direction between the inner tube and the outer tube.

**11.** A method according to claim 8 wherein the step of passing the sterilant through a circuitous path comprises passing the sterilant through at least one portion in which an effective cross-sectional area of the portion increases by at least 89% thereby decreasing the speed of the sterilant passing therethrough.

**12.** A method according to claim 8 wherein the step of passing the sterilant through the circuitous path comprises passing the sterilant through an orifice having a cross-sectional area no greater than 44.1% of a cross-sectional area of the circuitous path immediately upstream of the orifice.

**13.** A method according to claim 8 wherein the step of passing the sterilant through the circuitous path comprises having the sterilant make at least two turns, each of which are at least 90 degrees.

**14.** A method according to claim 8 wherein the non-vaporizable components comprise stabilizing compounds for the liquid phase of the sterilant.

**15.** A method according to claim 8 wherein the sterilant comprises hydrogen peroxide.

**16.** A method according to claim 8 wherein at least 75% of the non-vaporizable components are removed from the sterilant prior to the step of passing the sterilant out of the vaporizer.

**17.** A method according to claim 16 wherein substantially all of the non-vaporizable components are removed from the sterilant prior to the step of passing the sterilant out of the vaporizer.

**18.** A method according to claim 8 wherein the sterilant remains within the vaporizer for at least 17 milliseconds.

**19.** A method according to claim 18 wherein the sterilant remains within the vaporizer for at least 26 milliseconds.

**Patentansprüche**

**1.** Verdampfer (20) zum Verdampfen eines Sterilisiermittels aus seiner flüssigen Phase in einem Dampfphasen-Sterilisationssystem, das einen Druck unterhalb des Atmosphärendruckes hat, wobei der Verdampfer aufweist:

einen Einlaß (92), durch den das Sterilisiermittel in seiner flüssigen Phase erhalten wird;
einen Auslaß (88), durch den das Sterilisiermittel in seiner Dampfphase ausgelassen wird;
einen Umlaufweg zwischen dem Einlaß und dem Auslaß, um nicht verdampfbare Bestandteile des Sterilisiermittels zu sammeln; und
eine Strömungsdrossel (91, 116, 126, 128, 132) zwischen dem Umlaufweg und dem Auslaß, wobei die Drossel den Dampf innerhalb des Verdampfers für wenigstens 17 Millisekunden zurückhalten kann.

**2.** Verdampfer nach Anspruch 1, bei dem der Umlaufweg eine Vielzahl von Prallflächen (74) aufweist.

**3.** Verdampfer nach Anspruch 1, bei dem der Umlaufweg ein inneres Rohr (88, 116) aufweist, das konzentrisch innerhalb eines äußeren Rohres (68) angeordnet ist, wobei der Umlaufweg einen ersten Teil in einer ersten Richtung zwischen dem inneren Rohr und dem äußeren Rohr und einen zweiten Teil in einer zweiten entgegengesetzten Richtung durch das innere Rohr umfaßt.

**4.** Verdampfer nach Anspruch 1, bei dem der Umlaufweg wenigstens einen Teil aufweist, in dem eine wirksame Querschnittsfläche des Teiles um wenigstens 89 % zunimmt, um somit die Geschwindigkeit des durchlaufenden Sterilisiermittels abzusenken.

**5.** Verdampfer nach Anspruch 1, bei dem die Strömungsdrossel eine Öffnung (128, 132) aufweist, die eine Querschnittsfläche hat, welche nicht größer als 44.1 % einer Querschnittsfläche des Umlaufweges unmittelbar stromaufwärts der Öffnung ist.

**6.** Verdampfer nach Anspruch 1, bei dem der Umlaufweg wenigstens zwei Kehren aufweist, die jede wenigstens 90° betragen.

**7.** Verdampfer nach Anspruch 1, bei dem die Drossel den Dampf innerhalb des Verdampfers über wenigstens 26 Millisekunden zurückhalten kann.

**8.** Verfahren zum Liefern eines Sterilisiermittels in Dampfphase an eine Sterilisationskammer, das die Schritte aufweist:

Erzeugen von Temperatur- und Druckbedingungen innerhalb eines Verdampfers, die ausreichend sind, um das Sterilisiermittel zu verdampfen;
Eingeben des Sterilisiermittels in seiner flüssigen Phase in den Verdampfer und Verdampfen des Sterilisiermittels;
Leiten des Sterilisiermittels durch einen Umlaufweg und Sammeln nicht verdampfbarer Komponenten des Sterilisiermittels auf Oberflächen, welche den Umlaufweg bilden;
Leiten des Sterilisiermittels in seiner Dampfphase durch eine Strömungsdrossel; und
Herausleiten des Sterilisiermittels in seiner Dampfphase aus dem Verdampfer.

**9.** Verfahren nach Anspruch 8, bei dem der Schritt des Leitens des Sterilisiermittels durch einen Umlaufweg das Leiten des Sterilisiermittels an einer Vielzahl von Prallflächen vorbei aufweist.

**10.** Verfahren nach Anspruch 8, bei dem der Schritt des Leitens des Sterilisiermittels durch den Umlaufweg das Leiten des Sterilisiermittels in einer ersten Richtung durch ein inneres Rohr, das konzentrisch innerhalb eines äußeren Rohres angeordnet ist, und in eine zweite entgegengesetzte Richtung zwischen dem inneren Rohr und dem äußeren Rohr aufweist.

**11.** Verfahren nach Anspruch 8, bei dem der Schritt des Leitens des Sterilisiermittels durch einen Umlaufweg das Leiten des Sterilisiermittels durch wenigstens einen Teil, in dem eine effektive Querschnittsfläche des Teiles sich um wenigstens 89 % vergrößert, so daß die Geschwindigkeit des durchlaufenden Sterilisiermittels abgesenkt wird, aufweist.

**12.** Verfahren nach Anspruch 8, bei dem der Schritt des Leitens des Sterilisiermittels durch den Umlaufweg das Leiten des Sterilisiermittels durch eine Öffnung mit einer Querschnittsfläche, die nicht größer als 44.1 % einer Querschnittsfläche des Umlaufweges unmittelbar stromaufwärts der Öffnung ist, aufweist.

**13.** Verfahren nach Anspruch 8, bei dem der Schritt des Leitens des Sterilisiermittels durch den Umlaufweg das Zwingen des Sterilisiermittels in wenigstens zwei Kehren, die jede wenigstens 90° betragen, aufweist.

**14.** Verfahren nach Anspruch 8, bei dem die nicht verdampfbaren Komponenten stabilisierende Verbindungen für die flüssige Phase des Sterilisiermittels aufweisen.

**15.** Verfahren nach Anspruch 8, bei dem das Sterilisiermittel Wasserstoffperoxid aufweist.

**16.** Verfahren nach Anspruch 8, bei dem wenigstens 75 % der nicht verdampfbaren Komponenten vor dem Schritt des Leitens des Sterilisiermittels aus dem Verdampfer hinaus aus dem Sterilisiermittel entfernt werden.

**17.** Verfahren nach Anspruch 16, bei dem im wesentlichen die gesamten nicht verdampfbaren Komponenten vor dem Schritt des Leitens des Sterilisiermittels aus dem Verdampfer hinaus aus dem Sterilisiermittel entfernt werden.

**18.** Verfahren nach Anspruch 8, bei dem das Sterilisiermittel für wenigstens 17 Millisekunden innerhalb des Verdampfers verbleibt.

**19.** Verfahren nach Anspruch 18, bei dem das Sterilisiermittel für wenigstens 26 Millisekunden innerhalb des Verdampfers verbleibt.

**Revendications**

**1.** Vaporisateur (20) permettant de vaporiser un agent stérilisant à partir de sa phase liquide dans un système de stérilisation en phase vapeur ayant une pression inférieure à la pression atmosphérique, ledit vaporisateur comprenant :

une entrée (92) afin de recevoir l'agent stérilisant dans sa phase liquide ;
une sortie (88) afin de décharger l'agent stérilisant dans sa phase vapeur ;
un passage indirect entre l'entrée et la sortie afin de collecter des ingrédients non vaporisables de l'agent stérilisant ; et
un limitateur d'écoulement (91, 116, 126, 128, 132) entre le passage indirect et la sortie, moyennant quoi la limitation peut retenir la vapeur à l'intérieur du vaporisateur pendant au moins 17 millisecondes.

**2.** Vaporisateur selon la revendication 1, dans lequel le passage indirect comprend une pluralité de déflecteurs (74).

**3.** Vaporisateur selon la revendication 1, dans lequel le passage indirect comprend un tube interne (88,116) positionné de façon concentrique à l'intérieur d'un tube externe (68), le passage indirect comprenant une première partie dans une première direction entre le tube interne et le tube externe et une seconde partie dans une seconde direction opposée en passant par le tube interne.

**4.** Vaporisateur selon la revendication 1, dans lequel le passage indirect comprend au moins une partie dans laquelle une surface transversale effective de la partie augmente d'au moins 89 % afin de diminuer la vitesse de l'agent stérilisant qui passe à travers de celle-ci.

**5.** Vaporisateur selon la revendication 1, dans lequel la limitation d'écoulement comprend un orifice (128,132) ayant une surface transversale non supérieure à 44,1 % d'une surface transversale du passage indirect immédiatement en amont de l'orifice.

**6.** Vaporisateur selon la revendication 1, dans lequel le passage indirect comprend au moins deux virages, dont chacun est au moins de 90 degrés.

**7.** Vaporisateur selon la revendication 1, dans lequel la limitation peut retenir la vapeur à l'intérieur du vaporisateur pendant au moins 26 millisecondes.

**8.** Procédé permettant de proposer un agent stérilisant en phase vapeur à une chambre de stérilisation comprenant les étapes consistant à :

créer des conditions de température et de pression dans un vaporisateur, suffisantes pour vaporiser l'agent stérilisant ;
introduire l'agent stérilisant, dans sa phase liquide, dans le vaporisateur et vaporiser l'agent stérilisant ;
faire passer l'agent stérilisant par un passage indirect et collecter des composants non vaporisables de l'agent stérilisant sur les surfaces formant le passage indirect ;
faire passer l'agent stérilisant, dans sa phase vapeur, par un limitateur d'écoulement ; et
faire passer le stérilisant, dans sa phase vapeur, à l'extérieur du vaporisateur.

**9.** Procédé selon la revendication 8, dans lequel l'étape consistant à faire passer l'agent stérilisant par un passage indirect comprend l'étape consistant à faire passer l'agent stérilisant au-delà d'une pluralité de déflecteurs.

**10.** Procédé selon la revendication 8, dans lequel l'étape consistant à faire passer l'agent stérilisant par le passage indirect comprend l'étape consistant à faire passer l'agent stérilisant dans une première direction à travers un tube interne positionné de manière concentrique à l'intérieur d'un tube externe, et dans une seconde direction opposée entre le tube interne et le tube externe.

**11.** Procédé selon la revendication 8, dans lequel l'étape consistant à faire passer l'agent stérilisant par un passage indirect comprend l'étape consistant à faire passer le stérilisant par au moins une partie dans laquelle une surface transversale effective de la partie augmente d'au moins 89 %, diminuant ainsi la vitesse du stérilisant qui passe à travers celle-ci.

**12.** Procédé selon la revendication 8, dans lequel l'étape consistant à faire passer l'agent stérilisant par le circuit indirect comprend l'étape consistant à faire passer l'agent stérilisant par un orifice ayant une surface transversale non supérieure à 44,1 % de la surface transversale du passage indirect immédiatement en amont de l'orifice.

**13.** Procédé selon la revendication 8, dans lequel l'étape consistant à faire passer l'agent stérilisant par le passage indirect comprend le fait d'avoir l'agent stérilisant qui réalise au moins deux virages, dont chacun est d'au moins 90 degrés.

**14.** Procédé selon la revendication 8, dans lequel les composants non vaporisables comprennent des composés stabilisants pour la phase liquide de l'agent stérilisant.

**15.** Procédé selon la revendication 8, dans lequel l'agent stérilisant comprend du peroxyde d'hydrogène.

**16.** Procédé selon la revendication 8, dans lequel au moins 75 % des composants non vaporisables sont retirés de l'agent stérilisant avant l'étape consistant à faire sortir l'agent stérilisant du vaporisateur.

**17.** Procédé selon la revendication 16, dans lequel sensiblement tous les composants non vaporisables sont retirés de l'agent stérilisant avant l'étape consistant à faire sortir l'agent stérilisant du vaporisateur.

**18.** Procédé selon la revendication 8, dans lequel l'agent stérilisant reste dans le vaporisateur pendant au moins 17 millisecondes.

**19.** Procédé selon la revendication 18, dans lequel l'agent stérilisant reste dans le vaporisateur pendant au moins 26 millisecondes.

**FIG. 1**

PUMP 16

VALVE 18

RESERVOIR 14

STABILIZER 40

VAPORIZER 20

TUBE MANIFOLD 24

TUBE 4

CHAMBER 22

VALVE 28

VALVE 30

VACUUM PUMP 26

FIG. 2

EP 1 210 952 B1

FIG. 3

# FIG. 4

FIG. 5

FIG. 6

# FIG. 7

EP 1 210 952 B1

FIG. 8

EP 1 210 952 B1

*FIG. 9*

# FIG. 10

EP 1 210 952 B1

# FIG. 11

EP 1 210 952 B1